# EUROPEAN PATENT SPECIFICATION

(11) **EP 2 657 239 B1**
(45) Date of publication and mention of the grant of the patent: **27.05.2015**
(21) Application number: 10861197.1
(22) Date of filing: 20.12.2010
(51) Int. Cl.: C07D 495/14, C07C 13/567, C07C 13/573, C09K 11/06

(54) **ORGANIC SEMICONDUCTOR MATERIAL, PREPARATION METHODS AND USES THEREOF**
ORGANISCHES HALBLEITERMATERIAL, HERSTELLUNGSVERFAHREN UND ANWENDUNGEN DAVON
MATÉRIAU SEMI-CONDUCTEUR ORGANIQUE, SES PROCÉDÉS DE PRÉPARATION ET SES APPLICATIONS

(43) Date of publication of application: 30.10.2013
(73) Proprietor: Ocean's King Lighting Science&Technology Co., Ltd., Guangdong 518054 (CN)
(72) Inventor: ZHOU, Mingjie, Shenzhen, Guangdong 518054 (CN); HUANG, Jie, Shenzhen, Guangdong 518054 (CN); HUANG, Jiale, Shenzhen, Guangdong 518054 (CN)
(74) Representative: Michalski Hüttermann & Partner Patentanwälte mbB
(86) International application number: PCT/CN2010/079998
(87) International publication number: WO 2012/083514

(56) References cited:
- JP-A- 2003 128 651
- JP-A- 2003 229 273
- JP-A- 2010 087 406
- US-A1- 2008 262 183

## Description

### FIELD OF THE INVENTION

The present disclosure relates to organic semiconductor materials, and more particularly relates to an organic semiconductor material containing a fluorene unit. The present disclosure further relates to a preparation method and use of the organic semiconductor material.

### BACKGROUND OF THE INVENTION

The high-efficiency solar cells usually use inorganic semiconductors as raw material, however, current silicon solar cells have some disadvantages such as complex process of the production process, serious pollution, energy consumption, high cost, such that the development of their commercial applications is inhibited. Therefore the preparation of solar cells with low cost and high performance from the cheaper materials has been a research hotspot and difficulty of the photovoltaic field. The organic semiconductor material, on the one hand, exhibits a good environmental stability, low production cost, easy functional modulation, flexibility and better film forming properties, on the other hand, it has gained lots of concern due to the feature of relatively simple preparation process, low-temperature operation, and lower cost of device fabrication, such that it has become a cheap and attractive material for solar cells. Besides, the potential advantages of organic solar cells include: large area manufacture, flexible substrates can be used, environmentally friendly, lightweight and portable, etc..

Although the organic solar cells have been rapidly developed, but their conversion efficiency is still much lower than that of the inorganic solar cells. Major factors that constraints their performance are: the organic semiconductor device exhibits a relatively low carrier mobility, and spectral response of the device dose not match with the solar radiation spectrum, and red area with high photon flux is not be utilized effectively and electrode collection efficiency of carrier is low. In order to make the polymer solar cells be used in practical application, to develop new materials and to significantly improve the energy conversion efficiency are still the primary tasks of this research field.

Fluorene has a planar molecules characteristic and a relatively rigid structure, therefore it has a stable chemical properties. In addition, it can be introduced by other branched chain via a chemical reaction in the 9-position, and it can be easily cross-coupled to transition metal on 2, 7-position, therefore it has a good chemical modification. Furthermore, it also has a relatively wider band gap and lower the HOMO level, excellent quantum efficiency, a hole transport properties, and film-forming properties, therefore fluorene and its derivatives are widely used in the photovoltaic materials. Compounds for organic semiconductors are inter alia disclosed in the JP 2010 087406, US 2008/ 262 183, JP 2003-128651 and JP 2003-229273

### SUMMARY OF THE INVENTION

Based on the above problems, one object of the present invention is to provide an organic semiconductor material containing a fluorene unit.

An organic semiconductor material represented by the following general formula (P) is provided: 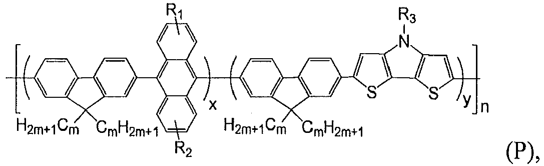 wherein:
R₁, R₂ are selected from hydrogen atom, fluorine atom, cyano group, alkyl or alkoxy that may be substituted or unsubstitued or aryl or heteroaryl that may be substituted or unsubstitued having a straight-chain or branched-chain of 1-40 carbon atoms; preferably, R₁, R₂ are selected from alkyl and alkoxy that may be substituted or unsubstitued having a straight-chain or branched-chain of 1-18 carbon atoms.
R₃ is selected from alkyl having 1-20 carbon atoms, preferably, R₃ is selected from alkyl having 6-12 carbon atoms;
n is a natural number and 1<n≤100;
m is a natural number and 1<m≤20; preferably 6≤m≤12.
x, y is a positive real number, and x + y = 1. x and y are determined by the feed ratio of the three reactants unit body, i.e. 2,7 - bis (4,4,5,5 - tetramethyl-1 ,3,2-dioxaborolan- yl) -9,9 - dialkyl-fluorenyl, 9,10 - dibromoanthracene or its derivatives, and dithiophene pyrrole or its derivative.

A preparation method of the organic semiconductor material is provided including the following steps:

Step S1, 2, 7 - dibromo-9, 9 - dialkyl fluorene and n-butyl lithium are dissolved in the first solvent according to a molar ratio of 1:2 to 1:4 at a temperature form -70°C to -85°C under an anhydrous and oxygen-free environment, then 2-isopropoxy-4, 4, 5, 5 - tetramethyl-1, 3, 2 - dioxaborolane or bis(pinacolato)diboron is added, the mixture is heated to a temperature form 20°C to 30°C and reacted for 12 to 48 hours to obtain 2, 7 - bis (4, 4, 1, 5, 5 - tetramethyl-1, 3, 2 - heteroaryl oxygen pentaborane alkyl) - 9, 9 - dialkyl fluorene;

Step S2, under an oxygen-free environment, 2, 7 - bis (4, 4, 1, 5, 5-tetramethyl-1, 3, 2 - heteroaryl oxygen pentaborane alkyl) - 9, 9 - dialkyl fluorene, 9, 10 - dibromoanthracene or derivatives thereof, and dithiophene pyrrole or derivatives thereof are added to a second solvent containing catalyst and an alkali solution according to a molar ratio of m:j:k, wherein m=j+k, Suzuki reaction is performed for 24 to 72 hours at a temperature form 70°C to 100°C, and the organic semiconductor material is obtained.

In step S1 of the method, the first solvent is tetrahydrofuran, diethyl ether, methylene chloride, chloroform or ethyl acetate. The molar amount of 2 - isopropoxy-4, 4, 5, 5 - tetramethyl-1, 3, 2 - dioxaborolane is 2 to 4 times of the molar amount of 2, 7-dibromo-9, 9 - dioxaneyl fluorene.

In step S2 of the method, the second solvent is at least one selected from the group consisting of benzene, chlorobenzene, toluene, ethylene glycol dimethyl ether, tetrahydrofuran, diethyl ether, methylene chloride, chloroform and ethyl acetate. The catalyst is organic palladium (e.g. Pd(PPh₃)₄, Pd(OAc)₂, Pd₂(dba)₃ or Pd(PPh₃)₂Cl₂) or a mixture of organic palladium and organophosphine ligand (e.g. tricyclohexylphosphine and P(o-Tol)₃), for example Pd₂(dba)₃ /P(o-Tol)₃). A molar amount of the catalyst is 0.0005 to 0.2 times of a molar amount of the 2, 7 - bis (4, 4, 5, 5 - tetramethyl-1, 3, 2-dioxaborolan -yl) - 9, 9 - dialkyl silafluorene. The alkali solution is NaOH aqueous solution, Na₂CO₃ aqueous solution, NaHCO₃ aqueous solution or tetraethyl ammonium hydroxide aqueous solution, and a molar amount of the alkali in the alkaline solution is 2 to 20 times of a molar amount of the 2, 7 - bis (4, 4, 5, 5 - tetramethyl-1, 3, 2-dioxaborolan -yl) - 9, 9 - dialkyl silafluorene.

The organic semiconductor material may be widely used in fields of organic solar cells, organic field-effect transistors, organic electroluminescent devices, organic optical memories, organic non-linear devices or organic laser devices.

Compared with the prior art, the present invention has at least the following advantages:
1. Since anthracene is introduced to the organic semiconductor material of the present invention, the carrier mobility of the material is significantly improved due to its good flatness and conjugation. Meanwhile, alkyl group, etc. can be easily introduced to the N atom of dithiophene pyrrole by modification, thus improving its solubility and processing performance;
2. Dithiophene pyrrole has a simple structure, the position of atoms in the molecule is relatively compact, and it has a symmetry structure. When it is used as a conjugated polymer unit, dithiophene pyrrole has a strong electron donating effect. Using dithiophene pyrrole unit and anthracene unit and fluorene unit to copolymerization, the band gap of the organic semiconductor material is effectively adjusted, so that the absorbance becomes strong and light absorption range become wide, thus improving the utilization of sunlight, and the same time, the excellent performance and the charge transport properties of the organic semiconductor material is realized.
3. The preparation method of the organic semiconductor material has the advantages of simple preparation process, mild reaction conditions, easy operation and control, and is suitable for industrialized production.

### BRIEF DESCRIPTION OF THE DRAWINGS

FIG. 1 is a schematic structure view of an organic solar cell device using the organic semiconductor material P1 of Example 5 according to the present invention as an active layer ;
FIG. 2 is an I-V curve of an organic solar cell device using the organic semiconductor material P1 of Example 5 according to the present invention as an active layer ;
FIG. 3 is a schematic structure view of an organic solar cell device using the organic semiconductor material P1 of Example 6 according to the present invention as an active layer ;
FIG. 4 is a schematic structure view of an organic solar cell device using the organic semiconductor material P1 of Example 7 according to the present invention as an active layer.

### DETAILED DESCRIPTION

An organic semiconductor material represented by the following general formula (P) is provided: 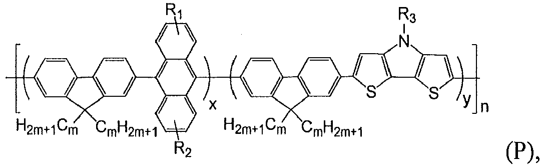 wherein:
R₁, R₂ are selected from hydrogen atom, fluorine atom, cyano group, alkyl or alkoxy that may be substituted or unsubstitued or aryl or heteroaryl that may be substituted or unsubstitued having a straight-chain or branched-chain of 1-40 carbon atoms; preferably, R₁, R₂ are selected from alkyl and alkoxy that may be substituted or unsubstitued having a straight-chain or branched-chain of 1-18 carbon atoms.
R₃ is selected from alkyl having 1-20 carbon atoms, preferably, R₃ is selected from alkyl having 6-12 carbon atoms;
n is a natural number and 1<n≤100;
m is a natural number and 1<m≤20; preferably 6≤m≤12.
x, y is a positive real number, and x + y = 1. x and y are determined by the feed ratio of the three reactants unit body, i.e. 2,7 - bis (4,4,5,5 - tetramethyl-1 ,3,2 - dioxaborolan-yl) -9,9 - dialkyl-fluorenyl, 9,10 - dibromoanthracene or its derivatives, and dithiophene pyrrole or its derivative.

A preparation method of the organic semiconductor material is provided including the following steps:

Step S1, 2, 7 - dibromo-9, 9 - dialkyl fluorene and n-butyl lithium (n-BuLi) are dissolved in the first solvent according to a molar ratio of 1.0:2.0 to 1.0:4.0 at a temperature form -70°C to -85°C under an anhydrous and oxygen-free environment, then 2 - isopropoxy-4, 4, 5, 5 - tetramethyl-1, 3, 2 - dioxaborolane (or bis(pinacolato)diboron, the mole amount of which is 2.0 to 4.0 times of that of the 2, 7 - dibromo-9, 9 - dialkyl fluorene) is added, the mixture is heated to a temperature form 20°C to 30°C and reacted for 12 to 48 hours to obtain the product, e.g. 2, 7 - bis (4, 4, 1, 5, 5 - tetramethyl-1, 3, 2 - heteroaryl oxygen pentaborane alkyl) - 9, 9 - dialkyl fluorene. The first solvent is tetrahydrofuran, diethyl ether, methylene chloride, chloroform or ethyl acetate, etc.. The reaction formula is shown as follows: 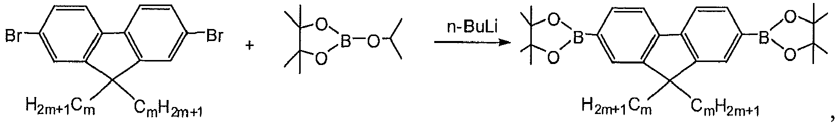 or 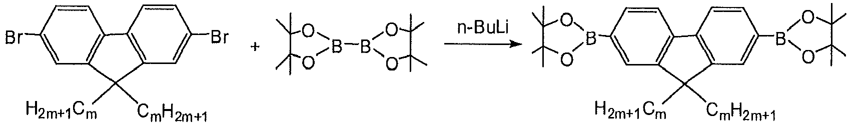 where m is a natural number and 1<m≤20; preferably 6≤m≤12.

Step S2, under an oxygen-free environment, 2, 7 - bis (4, 4, 1, 5, 5 - tetramethyl-1, 3, 2 - heteroaryl oxygen pentaborane alkyl) - 9, 9 - dialkyl fluorene, 9, 10 - dibromoanthracene or derivatives thereof, and dithiophene pyrrole or derivatives thereof are added to a second solvent containing catalyst and an alkali solution according to a molar ratio of m:j:k, wherein m=j+k, Suzuki reaction is performed for 24 to 72 hours at a temperature form 70°C to 100°C, and the organic semiconductor material is obtained. The catalyst is organic palladium (e.g. Pd(PPh₃)₄, Pd(OAc)₂, Pd₂(dba)₃ or Pd(PPh₃)₂Cl₂) or a mixture of organic palladium and organophosphine ligand (e.g. tricyclohexylphosphine and P(o-Tol)₃), for example Pd₂(dba)₃ /P(o-Tol)₃). A molar amount of the catalyst is 0.0005 to 0.2 times of a molar amount of the 2, 7 - bis (4, 4, 5, 5 - tetramethyl-1, 3, 2 - dioxaborolan -yl) - 9, 9 - dialkyl silafluorene. The alkali solution is NaOH aqueous solution, Na₂CO₃ aqueous solution, NaHCO₃ aqueous solution or tetraethyl ammonium hydroxide aqueous solution, and a molar amount of the alkali in the alkaline solution is 2 to 20 times of a molar amount of the 2, 7 - bis (4, 4, 5, 5 - tetramethyl-1, 3, 2 - dioxaborolan -yl) - 9, 9 - dialkyl silafluorene. The second solvent is benzene, chlorobenzene, toluene, ethylene glycol dimethyl ether, tetrahydrofuran, diethyl ether, methylene chloride, chloroform or ethyl acetate, etc.. The reaction formula is shown as follows: 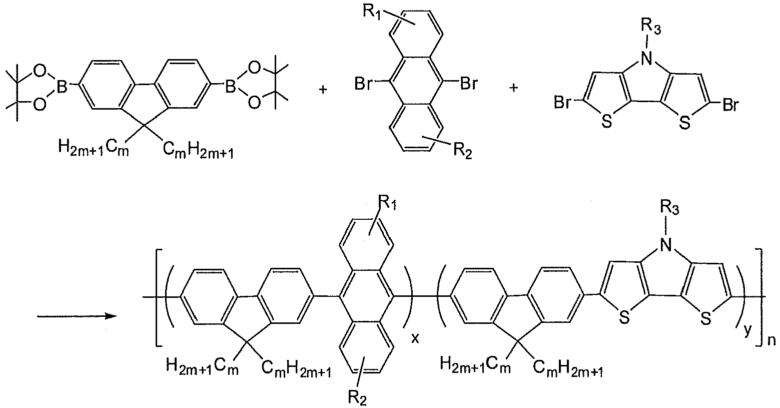 where,
R₁, R₂ are selected from hydrogen atom, fluorine atom, cyano group, alkyl or alkoxy that may be substituted or unsubstitued or aryl or heteroaryl that may be substituted or unsubstitued having a straight-chain or branched-chain of 1-40 carbon atoms; preferably, R₁, R₂ are selected from alkyl and alkoxy that may be substituted or unsubstitued having a straight-chain or branched-chain of 1-18 carbon atoms.
R₃ is selected from alkyl having 1-20 carbon atoms, preferably, R₃ is selected from alkyl having 6-12 carbon atoms.
n is a natural number and 1<n≤100.
x, y is a positive real number, and x + y = 1. x and y are determined by the feed ratio of the three reactants unit body.

Anthracene and its derivatives have good stability and good film-forming properties; their UV-visible spectroscopy shows a wide finger peak absorption, which help to improve the absorption range of sunlight. In addition, it has a appropriate carrier transporting characteristics, the hole mobility of its crystal at room temperature is up to 3 cm² /V•s, thus it is an excellent organic semiconductor material. Although there have been many reports on anthracene and its derivatives used as organic electroluminescent materials, but their usage as an organic photovoltaic material has rarely been reported, which greatly limits the scope of its application.

Dithiophene pyrrole has a simple structure, and its structural formula is shown below:

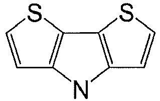

The position of atoms in the molecule is relatively compact, and the structure is symmetry, when used as a polymer unit of the conjugated organic semiconductor material, it also has a strong electron donating effect. Currently, dithiophene pyrrole compounds have become a popular material in the field of organic light research.

In the present invention, an organic semiconductor material containing a fluorene unit is provided, and it is used in organic solar cells and the like. This material has a lower energy gap, higher mobility, and a wide absorption range of the spectrum, and such material allows the carriers be transmitted more efficiently in the material of the active layer. After the organic solar cell prepared by the material according to the present invention as the active layer is high temperature annealed, the order and regularity of each group and molecular chain arranged in the molecule can effectively increase and improve the transmission speed and efficiency of the mobility of the carrier, thereby improving the photoelectric conversion efficiency.

To better understand the content of the present invention, the following specific examples and figures are described to further illustrate the technique of the invention, which includes the preparation and the uses of the organic semiconductor material in fields of organic solar cells, organic field-effect transistors, organic electroluminescent devices, organic optical memories, organic non-linear devices and organic laser devices, etc.

In the examples, the oxygen-free atmosphere forming the oxygen-free environment is primarily nitrogen atmosphere.

Example 1, this Example discloses the organic semiconductor material polymer P1, P2 with the following formula:

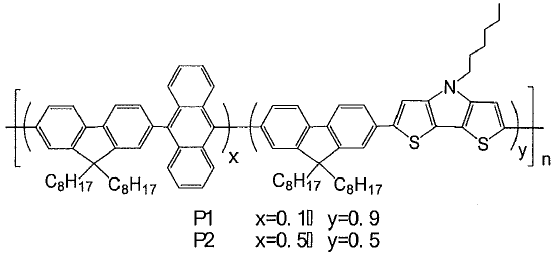

The preparation method of P1, P2 is described as follows:

Step one, preparation of 2,7 - bis (4,4,5,5 - tetramethyl-1 ,3,2-dioxaborolan- yl) -9,9 - dioctylfluorene:

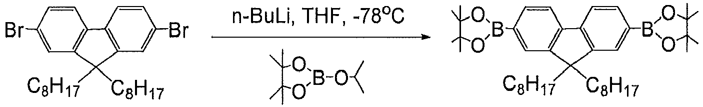

Anhydrous anaerobic reactor was assembled, under a continuing stirring and N₂ protection, white 9.0mmol of 2,7 - dibromo-9 ,9 - dioctylfluorene were added into a three-necked flask, 150mL of refined tetrahydrofuran solvent was added by a syringe, 27.0 mmol of n-BuLi was added by a syringe under a temperature of -78°C, the mixture was stirred for 2 hours. Then, 30.6mmol of 2 - isopropoxy-4, 4, 5, 5-tetramethyl-1, 3, 2 - dioxaborolane was added by a syringe under a temperature of -78°C, the temperature was raised to 20°C, and the reaction was carried out for 14 hours.

After the reaction was finished, a saturated aqueous NaCl solution was added, extracted with chloroform, dried by anhydrous sodium sulfate, suction filtered, and the filtrate was collected and solvent was rotary evaporated. The raw product was finally refined by a silica gel column chromatography using petroleum ether: ethyl acetate (v/v=15:1) as eluent to obtain powdered solid 2, 7 - bis (4,4,5,5-tetramethyl-1 ,3,2 - the dioxaborolan- yl) -9,9 - dioctylfluorene in a yield of 65%. GC-MS (EI-m/z): 642 (M⁺).

Step two, preparation of P1 and P2:

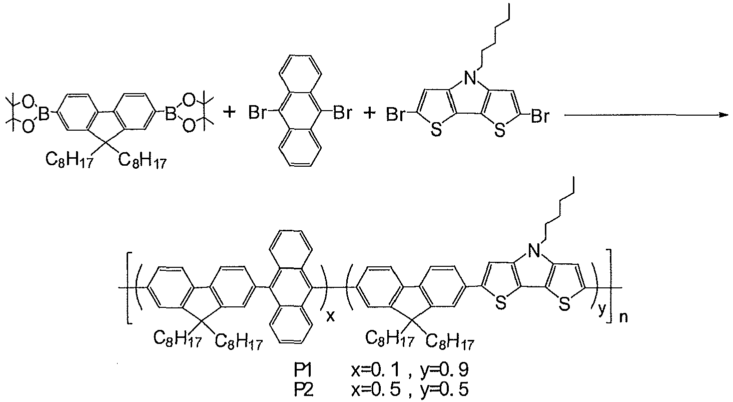

1mmol of 2,7 - bis (4,4,5,5 - tetramethyl-1,3,2 - dioxaborolan- yl) -9,9 - dioctylfluorene, 0.1mmol of 9,10 - dibromoanthracene, 0.9mmol of 2, 6-dibromo-4 - hexyl-dithiophene thieno pyrrolo, 0.025mmol of triphenylphosphine palladium, 5ml of 2mol/L Na₂CO₃ aqueous solution and 30ml toluene solvent were added to a reactor, the reaction system was kept in an oxygen-free environment by repeatedly introducing N₂ and vacuuming, the system was reacted for 72 hours at a temperature of 70°C.

72 hours later, deionized water and toluene were added to the reaction flask for extraction, the organic phase was separated, the organic semiconductor material polymer/toluene solution was distilled under reduce pressure to about 5 mL, and was added to 300mL of anhydrous methanol dropwise with constantly stirring, and a solid was precipitated. The solid was then filtrated, dried to obtain a solid powder. The solid powder was dissolved in chloroform, refined by neutral alumina column chromatography to remove the catalyst, and finally the organic semiconductor material polymer/chloroform solution was spin evaporated to the about 5mL, and added dropwise to the methanol solvent and stirred for several hours, and finally the organic semiconductor material polymer P1 was dried and collected. The organic semiconductor material was extracted with Soxhlet extractor, thereby improving the monodisperse polymer molecular weight of the organic semiconductor material.

Using Waters Breeze gel chromatography, the purified organic semiconductor material polymer P1 was dissolved in the refined tetrahydrofuran to form a 1mg/1mL solution. The insolubles were filtered off by supporting membrane for the instrument, then GPC test was carried out with 10 µL, a rate of 1mL/min. The number average molecular weight Mn≈64150, the polymer unit of the organic semiconductor material has a dispersion of 1.56, and n is 100.

1mmol of 2,7 - bis (4,4,5,5 - tetramethyl-1,3,2 - dioxaborolan- yl) -9,9 - dioctylfluorene, 0.5mmol of 9,10 - dibromoanthracene, 0.5mmol of 2,6 - dibromo-4 - hexyl - dithiophene thieno pyrrolo were added to a reactor, other feeding amount of the reactants, reaction conditions and post-treatment method were the same as that previously described, and the organic semiconductor material polymer P2 was obtained. Using Waters Breeze gel chromatography, the purified organic semiconductor material polymer P2 was dissolved in the refined tetrahydrofuran to form a 1mg/1mL solution. The insolubles were filtered off by supporting membrane for the instrument, then GPC test was carried out with 10 µL, a rate of 1mL/min. The number average molecular weight Mn≈39500, the polymer unit of the organic semiconductor material has a dispersion of 1.89, and n is 65.

Example 2, this Example discloses the organic semiconductor material polymer P3, P4 with the following formula:

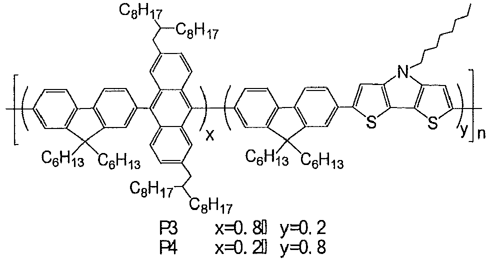

The preparation method of P3, P4 is described as follows:

Step one, preparation of 2,7 - bis (4,4,5,5-tetramethyl-1 ,3,2 - two miscellaneous oxopent borane) based -9,9 - diethyl fluorene:

The preparation process was similar to that of Step one in Example 1, and the differences were:

In step S1, the temperature was -75°C, and then was raised to 20°C. The reaction time was 48 hours. The mole ratio of 2,7-dibromo-9 ,9-dioctylfluorene to n-BuLi was 1:2; the solvent is dichloromethane; the mole amount of 2-isopropoxy-4, 4, 5, 5 - tetramethyl-1, 3, 2 - dioxaborolane was 3 times of that of 2,7 - dibromo-9,9 - dioctylfluorene.

Step three, preparation of P3 and P4:

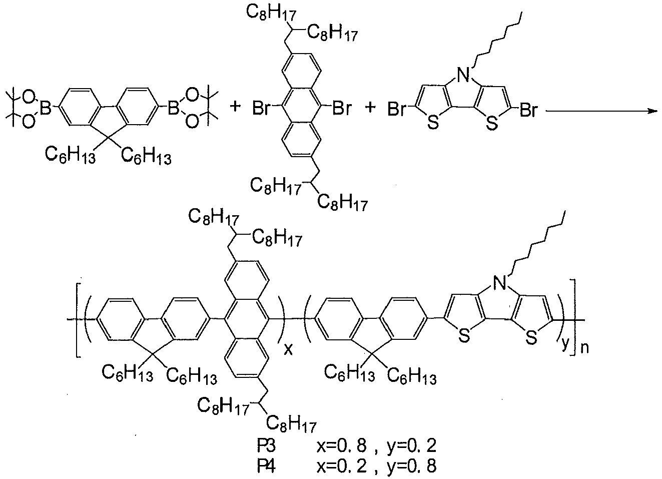

1mmol of 2,7 - bis (4,4,5,5 - tetramethyl-1 ,3,2 - two miscellaneous oxopent borane) based -9,9 - diethyl fluorene, 0.8mmol of 9,10 - dibromo-2 ,6 - two (2 - octyl decyl) anthracene (the synthesis method refer to Klaus Mullen et al, Macromol. Chem Phys. 2006, 207, 1107-1115), 0.2mmol of 2,6 - dibromo - 4 - octyl - dithiophene pyrrole, 3mg of palladium acetate, 10 ml of 2mol/L Na₂CO₃ aqueous solution and 40ml of toluene solvent were added to a reactor, the reaction system was kept in an oxygen-free environment by repeatedly introducing N₂ and vacuuming, the system was reacted for 24 hours at a temperature of 100°C.

24 hours later, deionized water and toluene were added to the reaction flask for extraction, the organic phase was separated, the organic semiconductor material polymer/toluene solution was distilled under reduce pressure to a small amount, and was added to 300mL of anhydrous methanol dropwise with constantly stirring, and a solid was precipitated. The solid was then filtrated, dried to obtain a solid powder. The solid powder was dissolved in chloroform, refined by neutral alumina column chromatography to remove the catalyst, and finally the organic semiconductor material polymer/chloroform solution was spin evaporated to the about 5mL, and added dropwise to the methanol solvent and stirred for several hours, and finally the organic semiconductor material polymer P3 was dried and collected. The organic semiconductor material was extracted with Soxhlet extractor, thereby improving the monodisperse polymer molecular weight of the organic semiconductor material.

Using Waters Breeze gel chromatography, the purified organic semiconductor material polymer P3 was dissolved in the refined tetrahydrofuran to form a 1mg/1mL solution. The insolubles were filtered off by supporting membrane for the instrument, then GPC test was carried out with 10 µL, a rate of 1mL/min. The number average molecular weight Mn≈35500, the polymer unit of the organic semiconductor material has a dispersion of 2.04, and n is 37.

1mmol of 2,7 - bis (4,4,5,5 - tetramethyl-1,3,2-dioxaborolan- yl) -9,9 - dihexylfluorene, 0.2mmol of 9,10 - dibromo-2 ,6 - two (2 - octyl decyl) anthracene, 0.8mmol of 2,6 - dibromo - 4 - octyl - dithiophene pyrrole were added to a reactor, other feeding amount of the reactants, reaction conditions and post-treatment method were the same as that previously described, and the organic semiconductor material polymer P4 was obtained. Using Waters Breeze gel chromatography, the purified organic semiconductor material polymer P4 was dissolved in the refined tetrahydrofuran to form a 1mg/1mL solution. The insolubles were filtered off by supporting membrane for the instrument, then GPC test was carried out with 10 µL, a rate of 1mL/min. The number average molecular weight Mn≈30100, the polymer unit of the organic semiconductor material has a dispersion of 2.10, and n is 44.

Example 3, this Example discloses the organic semiconductor material polymer P5, P6 with the following formula:

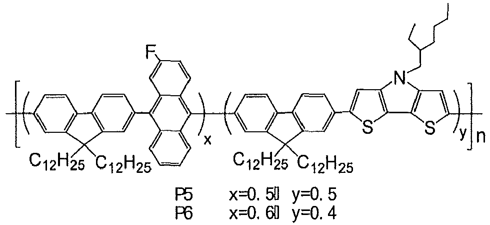

The preparation method of P5, P6 is described as follows:

Step one, preparation of 2,7 - bis (4,4,5,5-tetramethyl-1 ,3,2 - dioxaborolan-yl) -9,9 - bis (dodecyl) fluorene:

The preparation process was similar to that of Step one in Example 1, and the differences were:

In step S1, the temperature was -85°C, and then was raised to 25°C. The reaction time was 32 hours. The mole ratio of 2, 7-dibromo-9, 9-bis (dodecyl) fluorene to n-BuLi was 1:4; the solvent is ether; the mole amount of 2-isopropoxy-4, 4, 5,5-tetramethyl-1,3, 2 - dioxaborolane was 2 times of that of 2, 7 - dibromo-9,9 - bis (dodecyl) fluorene.

Step two, preparation of P5 and P6:

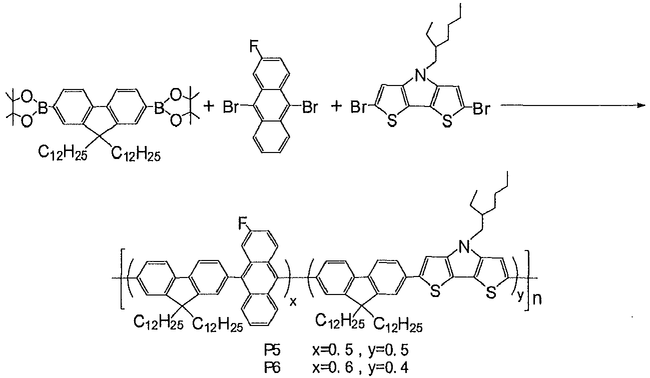

1mmol of 2,7-bis (4,4,5,5 - tetramethyl-1,3,2 - dioxaborolan- yl) -9,9 - bis (dodecyl) fluorene, 0.5mmol of 9,10 - dibromo-2-fluoroanthracene (the synthesis method refer to Elimelech Rochlin et al, J.Org.Chem., 2003, 68, 216-226), 0.5mmol of 2,6-dibromo-4 - (2'-ethyl-hexyl)-dithiophene pyrrole, 0.02mmol of triphenylphosphine palladium, 10 ml of 2mol/L Na₂CO₃ aqueous solution and 40ml of toluene solvent were added to a reactor, the reaction system was kept in an oxygen-free environment by repeatedly introducing N₂ and vacuuming, the system was reacted for 58 hours at a temperature of 80°C.

58 hours later, deionized water and toluene were added to the reaction flask for extraction, the organic phase was separated, the organic semiconductor material polymer/toluene solution was distilled under reduce pressure to a small amount, and was added to 300mL of anhydrous methanol dropwise with constantly stirring, and a solid was precipitated. The solid was then filtrated, dried to obtain a solid powder. The solid powder was dissolved in chloroform, refined by neutral alumina column chromatography to remove the catalyst, and finally the organic semiconductor material polymer/chloroform solution was spin evaporated to the about 5mL, and added dropwise to the methanol solvent and stirred for several hours, and finally the organic semiconductor material polymer P5 was dried and collected. The organic semiconductor material was extracted with Soxhlet extractor, thereby improving the monodisperse polymer molecular weight of the organic semiconductor material.

Using Waters Breeze gel chromatography, the purified organic semiconductor material polymer P5 was dissolved in the refined tetrahydrofuran to form a 1mg/1mL solution. The insolubles were filtered off by supporting membrane for the instrument, then GPC test was carried out with 10 µL, a rate of 1mL/min. The number average molecular weight Mn≈18000, the polymer unit of the organic semiconductor material has a dispersion of 2.41, and n is 26.

1mmol of 2,7 - bis (4,4,5,5 - tetramethyl-1 ,3,2 - dioxaborolan- yl) -9,9 - bis (dodecyl) fluorene, 0.6mmol of 9,10 - dibromo-2 - fluoroanthracene, 0.4mmol of 2,6 - dibromo-4 - (2'-ethyl-hexyl) - dithiophene pyrrole were added to a reactor, other feeding amount of the reactants, reaction conditions and post-treatment method were the same as that previously described, and the organic semiconductor material polymer P6 was obtained. Using Waters Breeze gel chromatography, the purified organic semiconductor material polymer P6 was dissolved in the refined tetrahydrofuran to form a 1mg/1mL solution. The insolubles were filtered off by supporting membrane for the instrument, then GPC test was carried out with 10 µL, a rate of 1mL/min. The number average molecular weight Mn≈20000, the polymer unit of the organic semiconductor material has a dispersion of 2.74, and n is 28.

Example 4, this Example discloses the organic semiconductor material polymer P7, P8 with the following formula:

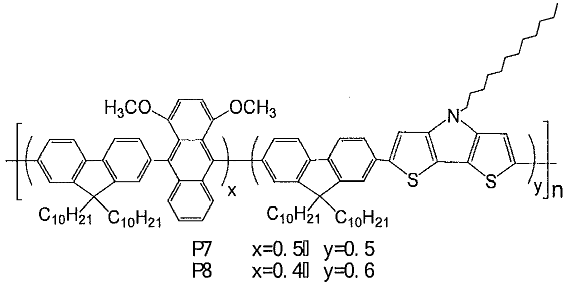

The preparation method of P7, P8 is described as follows:

Step one, preparation of 2,7-bis (4,4,5,5-tetramethyl-1 ,3,2 - dioxaborolan- yl) -9,9 - didecyl fluorenyl:

The preparation process was similar to that of Step one in Example 1, and the differences were:

In step S1, the temperature was -80°C, and then was raised to 30°C. The reaction time was 36 hours. The mole ratio of 2, 7 - dibromo-9, 9 - two decyl fluorenyl to n-BuLi was 1:2.5; the solvent is chloroform; the mole amount of 2-isopropoxy-4, 4, 5, 5 - tetramethyl-1, 3, 2 - dioxaborolane was 4 times of that of 2, 7 - dibromo-9,9 - two decyl fluorenyl.

Step two, preparation of P7 and P8:

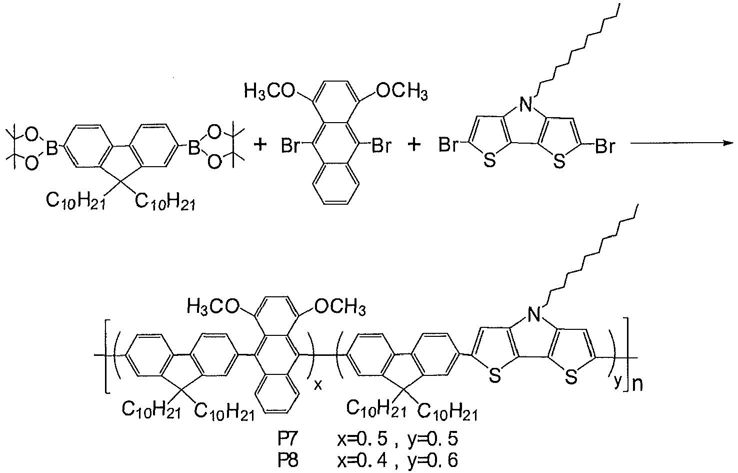

1mmol of 2,7 - bis (4,4,5,5 - tetramethyl-1,3,2-dioxaborolan- yl) -9,9 - didecyl fluorenyl, 0.5mmol of 9,10 - dibromo-1 ,4-dimethoxyanthracene (the synthesis method refer to Osman Cakmak et al J.Org.Chem., 2006, 71, 1795-1801), 0.5mmol of 2,6 - dibromo-4-Twelve alkyl - dithiazine of thiophene and pyrrole, 5mg of tricyclohexylphosphine, 10 ml of 2mol/L Na₂CO₃ aqueous solution and 50ml of toluene solvent were added to a reactor, the reaction system was kept in an oxygen-free environment by repeatedly introducing N₂ and vacuuming, the system was reacted for 64 hours at a temperature of 90°C.

64 hours later, deionized water and toluene were added to the reaction flask for extraction, the organic phase was separated, the organic semiconductor material polymer/toluene solution was distilled under reduce pressure to a small amount, and was added to 300mL of anhydrous methanol dropwise with constantly stirring, and a solid was precipitated. The solid was then filtrated, dried to obtain a solid powder. The solid powder was dissolved in chloroform, refined by neutral alumina column chromatography to remove the catalyst, and finally the organic semiconductor material polymer/chloroform solution was spin evaporated to the about 5mL, and added dropwise to the methanol solvent and stirred for several hours, and finally the organic semiconductor material polymer P7 was dried and collected. The organic semiconductor material was extracted with Soxhlet extractor, thereby improving the monodisperse polymer molecular weight of the organic semiconductor material.

Using Waters Breeze gel chromatography, the purified organic semiconductor material polymer P7 was dissolved in the refined tetrahydrofuran to form a 1mg/1mL solution. The insolubles were filtered off by supporting membrane for the instrument, then GPC test was carried out with 10 µL, a rate of 1mL/min. The number average molecular weight Mn≈53000, the polymer unit of the organic semiconductor material has a dispersion of 1.91, and n is 72.

1mmol of 2,7 - bis (4,4,5,5 - tetramethyl-1 ,3,2-dioxaborolan- yl) -9,9 - didecyl fluorenyl, 0.4mmol of 9,10 - dibromo-1 ,4 - dimethoxyanthracene, 0.6mmol of 2,6-dibromo-4 - Twelve alkyl-dithiazine of thiophene and pyrrole were added to a reactor, other feeding amount of the reactants, reaction conditions and post-treatment method were the same as that previously described, and the organic semiconductor material polymer P8 was obtained. Using Waters Breeze gel chromatography, the purified organic semiconductor material polymer P8 was dissolved in the refined tetrahydrofuran to form a 1mg/1mL solution. The insolubles were filtered off by supporting membrane for the instrument, then GPC test was carried out with 10 µL, a rate of 1mL/min. The number average molecular weight Mn≈44800, the polymer unit of the organic semiconductor material has a dispersion of 2.17, and n is 60.

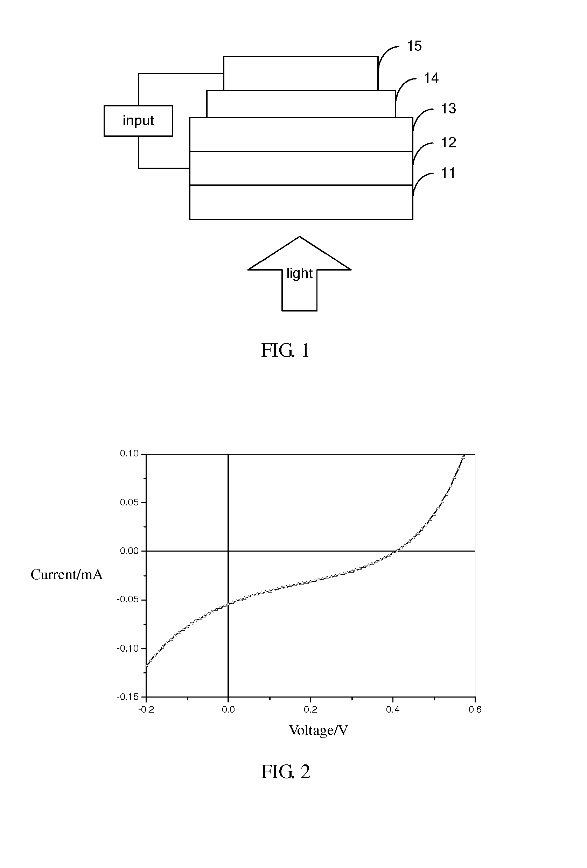

Example 5, preparation of an organic solar cell device using the organic semiconductor material P1 of Example 1 as an active layer material, the structure of which is shown in FIG. 1.

The structure of the organic solar cell device was: glass 11/ITO layer 12/PEDOT: PSS layer 13/active layer 14/Al layer 15; where the material of the active layer 14 contains the electron donor material and electron acceptor material. The electron donor material was the organic semiconductor material P1 in Example 1, [6,6]-phenyl-C₆₁-butyric acid methyl ester (PCBM) was used as the electron acceptor material. ITO was indium tin oxide with a sheet resistance of 10-20 Ω /sq, PEDOT was poly (3,4 - ethylenedioxy-thiophene), PSS was poly (styrene sulfonic acid); after ultrasonic washing of the ITO glass, an oxygen-Plasma processing was performed, and PEDOT: PSS was spin-coated on the ITO.

The organic semiconductor material in the present invention as an electron donor material and PCBM as an electron acceptor material were prepared by spin coating, the aluminum electrode was prepared by vacuum deposition techniques, and the organic solar cell device was obtained. The organic solar cell device was encapsulated using an epoxy resin, was annealed for 2 hours in 110°C sealed conditions, and then cooled to room temperature. After annealing of the organic solar cell device, the chemical structure of the material became more regular and orderly, the transmission speed and efficiency of the carrier were improved, thereby improving the photoelectric conversion efficiency of the organic solar cell device.

The material P1 in Example 1 was taken as an example, the thickness of the ITO layer, PEDOT: PSS layer, the active layer, the A1 layer were 110 nm, 40 nm, 110 nm, 110 nm, respectively. The prepared cell had an effective area of 9 mm². The measurement was conducted under the solar simulator, the intensity of light was verified with the silicon standard battery, IV curves were measured with a Keithley 2400. The IV curve of the device at 100 milliwatts per square centimeter of the analog light conditions was shown in FIG. 2. The open circuit voltage was 0.41 volts, the short-circuit current was 0.055 mA, and the fill factor was 0.46, the energy conversion efficiency was 0.12%.

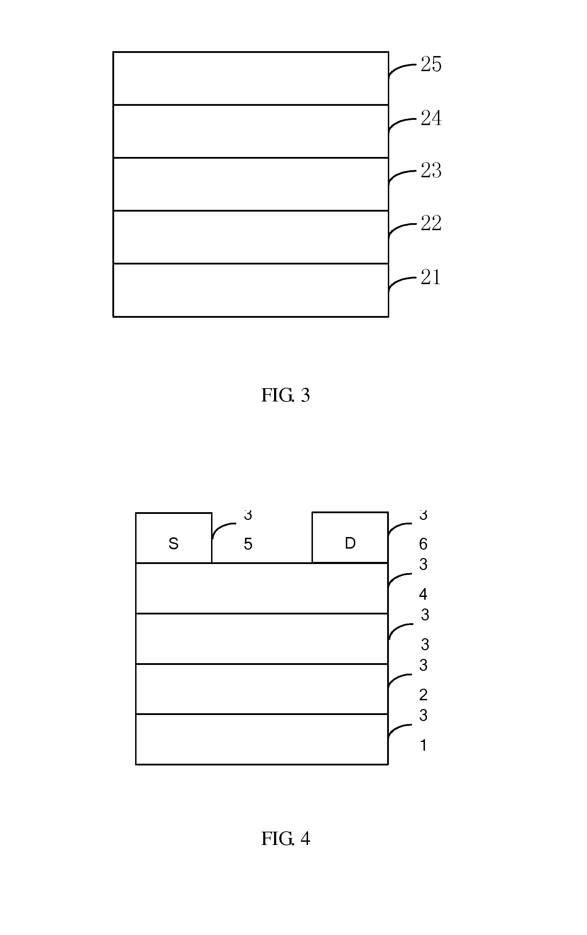

Example 6, preparation of an organic electroluminescent device using the organic semiconductor material P1 of Example 1 as the light emitting layer, the structure of which is shown in FIG. 3.

The structure of the organic electroluminescent device was: an indium tin oxide layer 22 with sheet resistance of 10-20Ω/sq was deposited on a glass substrate 21 as a transparent anode; the light emitting layer 23 was prepared on the ITO layer 22 by spin coating technique using the organic semiconductor material P1 in Example 1; LiF was vacuum deposited on this light emitting layer 23 as the buffer layer 24, the Al layer 25 was finally deposited as the cathode of the device.

Example 7, preparation of an organic field effect transistor using the organic semiconductor material P1 of Example 1 as the light emitting layer, the structure of which is shown in FIG. 4.

An organic field effect transistors used silicon (Si) as the substrate 31, SiO₂ with a thickness of 450 nm as the insulating layer 32, the organic semiconductor layer 34 with the organic semiconductor material of the present invention was pin-coated on an octadecyltrichlorosilane (OTS) layer 33 for modifying the SiO₂ layer 32; gold (and other metal material, aluminum, platinum, silver) may also be used as the electrode source electrode (S) 35 and the drain electrode (D) 36 on the organic semiconductor layer 34, and the organic field effect transistor containing P1 was obtained.

## Claims

1. An organic semiconductor material, represented by the following general formula (P): 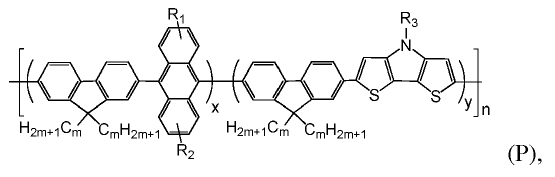 wherein:
R₁, R₂ are selected from hydrogen atom, fluorine atom, cyano group, alkyl or alkoxy or aryl or heteroaryl having a straight-chain or branched-chain of 1-40 carbon atoms; R₃ is selected from alkyl having 1-20 carbon atoms;
n is a natural number and 1<n≤100; m is a natural number and 1<m≤20; x, y is a positive real number, and x + y = 1.

2. The organic semiconductor material according to claim 1, wherein R₁, R₂ are selected from alkyl and alkoxy having a straight-chain or branched-chain of 1-18 carbon atoms.

3. The organic semiconductor material according to claim 1, wherein R₃ is selected from alkyl having 6-12 carbon atoms.

4. The organic semiconductor material according to claim 2, wherein 6≤m≤12.

5. A preparation method of the organic semiconductor material according to any one of claims 1 to 4, comprising the following steps:
S1, dissolving 2, 7 - dibromo-9, 9 - dialkyl fluorene and n-butyl lithium in the first solvent according to a molar ratio of 1:2 to 1:4 at a temperature form -70°C to -85°C under an anhydrous and oxygen-free environment, then adding 2 - isopropoxy-4, 4, 5, 5 - tetramethyl-1, 3, 2 - dioxaborolane or bis(pinacolato)diboron, heating to a temperature form 20°C to 30°C, reacting for 12 to 48 hours to obtain 2, 7 - bis (4, 4, 1, 5, 5 - tetramethyl-1, 3, 2 - heteroaryl oxygen pentaborane alkyl)-9, 9 - dialkyl fluorene; and
S2, adding 2, 7 - bis (4, 4, 1, 5, 5 - tetramethyl-1, 3, 2 - heteroaryl oxygen pentaborane alkyl) - 9, 9 - dialkyl fluorene, 9, 10 - dibromoanthracene or derivatives thereof, and dithiophene pyrrole or derivatives thereof to a second solvent containing catalyst and an alkali solution according to a molar ratio of m:j:k, wherein m=j+k, under an oxygen-free environment, performing Suzuki reaction for 24 to 72 hours at a temperature form 70°C to 100°C, and obtaining the organic semiconductor material.

6. The preparation method according to claim 5, wherein in step S1, the first solvent is at least one selected from the group consisting of tetrahydrofuran, diethyl ether, methylene chloride, chloroform and ethyl acetate; the molar amount of 2 - isopropoxy-4, 4, 5, 5 - tetramethyl-1, 3, 2-dioxaborolane is 2 to 4 times of the molar amount of 2, 7 - dibromo-9, 9 - dioxaneyl fluorene.

7. The preparation method according to claim 5, wherein in step S2, the second solvent is at least one selected from the group consisting of benzene, chlorobenzene, toluene, ethylene glycol dimethyl ether, tetrahydrofuran, diethyl ether, methylene chloride, chloroform and ethyl acetate.

8. The preparation method according to claim 5 or claim 7, wherein in step S2,
the catalyst is organic palladium or a mixture of organic palladium and organophosphine ligand, a molar amount of the catalyst is 0.0005 to 0.2 times of a molar amount of the 2, 7 - bis (4, 4, 5, 5 - tetramethyl-1, 3, 2 - dioxaborolan -yl) - 9, 9 - dialkyl silafluorene;
the alkali solution is at least one selected from the group consisting NaOH aqueous solution, Na₂CO₃ aqueous solution, NaHCO₃ aqueous solution and tetraethyl ammonium hydroxide aqueous solution, a molar amount of the alkali in the alkaline solution is 2 to 20 times of a molar amount of the 2, 7 - bis (4, 4, 5, 5-tetramethyl-1, 3,2-dioxaborolan -yl)-9, 9 - dialkyl silafluorene.

9. The preparation method according to claim 8, wherein the organic palladium is at least one selected from the group consisting Pd(PPh₃)₄, Pd(OAc)₂, Pd₂(dba)₃ and Pd(PPh₃)₂Cl₂; and the organophosphine ligand is tricyclohexylphosphine or P(o-Tol)₃, the mixture of the organic palladium and the organophosphine ligand is Pd₂(dba)₃/ P(o-Tol)₃.

10. Uses of the organic semiconductor material according to any one of claims 1 to 4 in fields of organic solar cells, organic field-effect transistors, organic electroluminescent devices, organic optical memories, organic non-linear devices or organic laser devices, etc..

## Patentansprüche

1. Ein organisches Halbleitermaterial, repräsentiert durch die folgende generelle Formel (P): 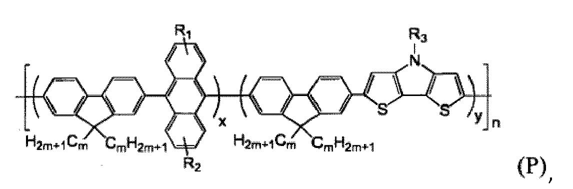 wobei:
R₁, R₂ ausgesucht sind aus Wasserstoff-Atom, Fluor-Atom, Cyano-Gruppe, Alkyl oder Alkoxy oder Aryl oder Heteroaryl aufweisend eine unverzweigte oder verzweigte Kette aus 1-40 Kohlenstoffatomen; R₃ ist ausgesucht aus Alkyl aufweisend 1-20 Kohlenstoffatome;
n ist eine natürliche Zahl und 1 < n ≤ 100; m ist eine natürliche Zahl und 1 < m ≤ 20; x, y ist eine positive reelle Zahl, und x + y = 1.

2. Das organische Halbleitermaterial nach Anspruch 1, wobei R₁, R₂ ausgewählt sind aus Alkyl und Alkoxy mit einer verzweigten oder unverzweigten Kette aus 1-18 Kohlenstoff-Atomen.

3. Das organische Halbleitermaterial nach Anspruch 1, wobei R₃ ausgewählt ist aus Alkyl mit 6-12 Kohlenstoff-Atomen.

4. Das organische Halbleitermaterial nach Anspruch 2, wobei 6 ≤ m ≤ 12.

5. Ein Verfahren zur Herstellung des organischen Halbleitermaterials nach einem der Ansprüche 1 bis 4, aufweisend die folgenden Schritte:
S1 Auflösen von 2,7-dibromo-9, 9-dialkylfluoren und n-butyl-Lithium im ersten Lösungsmittel mit einem molaren Verhältnis von 1:2 bis zu 1:4 bei einer Temperatur von -70°C bis zu -85°C in einer wasser- und sauerstofffreien Umgebung, anschließend Hinzufügen von 2-isopropoxy-4, 4, 5, 5-tetramethyl-1, 3, 2-dioxaborolan oder bis(pinacolato)dibor, Erhitzen auf eine Temperatur von 20°C bis zu 30°C, für 12 bis zu 48 Stunden reagieren lassen um 2, 7-bis (4, 4, 1, 5, 5- tetramethyl-1, 3, 2 - heteroaryl oxo pentaboran alkyl) - 9, 9-dialkyl fluoren zu erhalten; und
S2, Hinzufügen von 2, 7-bis(4, 4, l, 5, 5-tetramethyl-1, 3, 2- heteroaryl oxo pentaboran alkyl)-9, 9-dialkyl fluoren, 9, 10-dibromoanthracen oder Derivaten davon, und Dithiophenpyrrol oder Derivaten davon zu einem zweiten Lösungsmittel aufweisend den Katalysator und eine Alkali-Lösung mit einem molaren Verhältnis von m:j:k, wobei m=j+k, in einer sauerstofffreien Umgebung, Ausführen der Suzuki-reaktion für 24 bis zu 72 Stunden bei einer Temperatur von 70°C bis zu 100°C, und derart das organische Halbleitermaterial zu erhalten.

6. Das Verfahren nach Anspruch 5, wobei in Schritt S1 das erste Lösungsmittel ausgewählt ist aus der Gruppe bestehend aus Tetrahydrofuran, Diethylether, Methylenchlorid, Chloroform und Ethylacetat; die molare Menge von 2-isopropoxy-4, 4, 5, 5- tetramethyl-1, 3, 2-dioxaborolan beträgt 2 bis 4 mal die molare Menge von 2, 7-dibromo-9, 9-dioxaneyl fluoren.

7. Das Verfahren nach Anspruch 5, wobei in Schritt S2 das zweite Lösungsmittel wenigstens eines ausgewählt aus der Gruppe bestehend aus Benzol, Chlorbenzol, Toluol, Ethylenglykoldimethylether, Tetrahydrofuran, Diethylether, Methylenchlorid, Chloroform und Ethylacetat ist.

8. Das Verfahren nach Anspruch 5 oder Anspruch 7, wobei in Schritt S2 der Katalysator organisches Palladium oder eine Mischung von organischem Palladium und einem organophosphin-Liganden ist, die molare Menge des Katalysators beträgt das 0,0005 bis zu 0,2 fache der molaren Menge an 2,7-bis (4, 4, 5, 5-tetramethyl-1, 3, 2 - dioxaborolan-yl)-9, 9-dialkyl silafluoren; die Alkali-Lösung ist wenigstens eine ausgesucht aus der Gruppe bestehend aus wässriger NaOH-Lösung, wässriger Na₂CO₃-Lösung und wässriger Tetraethylammoniumhydroxid-Lösung, die molare Menge des Alkali in der Alkali-Lösung beträgt das 2 bis 20fache der molaren Menge an 2, 7-bis (4, 4, 5, 5-tetramethyl-1, 3, 2 - dioxaborolan-yl)-9, 9-dialkyl silafluoren.

9. Das Verfahren nach Anspruch 8, wobei das organische Palladium wenigstens eines ausgesucht aus der Gruppe bestehend aus Pd(PPh₃)₄, Pd(OAc)₂, Pd₂(dba)₃ und Pd(PPh₃)₂Cl₂ ist; und der organophosphin-Ligand ist tricyclohexylphosphin oder P(o-Tol)₃, die Mischung des organischen Palladiums und des organophosphin-Liganden ist Pd₂(dba)₃/P(o-Tol)₃.

10. Verwendung des organischen Halbleitermaterials nach einem der Ansprüche 1 bis 4 in den Gebieten organischer Solarzellen, organischer Feld-Effekt Transistoren, organischer elektrolumineszierender Geräte, organischen optischen Speichern, organischen nichtlinearen Geräten oder organischen Laser-Geräten, etc..

## Revendications

1. Matériau semi-conducteur organique, représenté par la formule générale suivante (P): 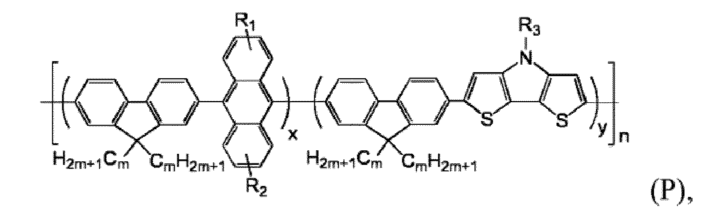 dans lequel:
R₁, R₂ sont choisis parmi un atome d'hydrogène, un atome de fluor, un groupe cyano, un alkyle ou un alcoxy ou un aryle ou un hétéroaryle ayant une chaîne linéaire ou une chaîne ramifiée de 1 à 40 atomes de carbone ; R₃ est choisi parmi un alkyle ayant 1 à 20 atomes de carbone ;
n est un nombre entier naturel et 1<n≤100 ; m est un nombre entier naturel et 1<m≤20 ; x, y est un nombre réel positif, et x + y = 1.

2. Matériau semi-conducteur organique selon la revendication 1, dans lequel R₁, R₂ sont choisis parmi un alkyle et un alcoxy ayant une chaîne linéaire ou une chaîne ramifiée de 1 à 18 atomes de carbone.

3. Matériau semi-conducteur organique selon la revendication 1, dans lequel R₃ est choisi parmi un alkyle ayant 6 à 12 atomes de carbone.

4. Matériau semi-conducteur organique selon la revendication 2, dans lequel 6≤m≤12.

5. Procédé de préparation du matériau semi-conducteur organique selon l'une quelconque des revendications 1 à 4, comprenant les étapes suivantes:
S1, la dissolution de 2,7-dibromo-9,9-dialkyl fluorène et de n-butyl lithium dans le premier solvant selon un rapport molaire de 1:2 à 1:4 à une température de -70°C à -85°C sous un environnement anhydre et exempt d'oxygène, puis l'ajout de 2-isopropoxy-4,4,5,5-tétraméthyl-1,3,2-dioxaborolane ou bis(pinacolato)dibore, le chauffage à une température de 20°C à 30°C, la réaction pendant 12 à 48 heures pour obtenir du 2,7-bis(4,4,1,5,5-tétraméthyl-1,3,2-hétéroaryle-oxopentyle de bore alcoylé)-9,9-dialkyl-fluorène ; et
S2, l'ajout de 2,7-bis(4,4,1,5,5-tétraméthyl-1,3,2-hétéroaryle-oxopentyle de bore alcoylé)-9,9-dialkyl-fluorène, de 9,10-dibromoanthracène ou de dérivés de celui-ci, et de pyrrole de dithiophène ou de dérivés de celui-ci à un deuxième solvant contenant un catalyseur et une solution alcaline selon un rapport molaire de m:j:k, dans lequel m=j+k, dans un environnement exempt d'oxygène, la réalisation d'une réaction de Suzuki pendant 24 à 72 heures à une température de 70°C à 100°C, et l'obtention du matériau semi-conducteur organique.

6. Procédé de préparation selon la revendication 5, dans lequel dans l'étape S1, le premier solvant est au moins un solvant choisi dans le groupe constitué par le tétrahydrofurane, l'éther diéthylique, le chlorure de méthylène, le chloroforme et l'acétate d'éthyle ; la quantité molaire de 2-isopropoxy-4,4,5,5-tétraméthyl-1,3,2-dioxaborolane est de 2 à 4 fois la quantité molaire de 2,7-dibromo-9,9-dioxaneyl fluorène.

7. Procédé de préparation selon la revendication 5, dans lequel dans l'étape S2, le deuxième solvant est au moins un solvant choisi dans le groupe constitué par le benzène, le chlorobenzène, le toluène, l'éthylène glycol dimethyl éther, le tétrahydrofurane, l'éther diéthylique, le chlorure de méthylène, le chloroforme et l'acétate d'éthyle.

8. Procédé de préparation selon la revendication 5 ou la revendication 7, dans lequel dans l'étape S2,
le catalyseur est un palladium organique ou un mélange d'un palladium organique et d'un ligand organophosphine, une quantité molaire du catalyseur est de 0,0005 à 0,2 fois la quantité molaire du 2,7-bis(4,4,5,5-tétraméthyl-1,3,2-dioxaborolan-yl)-9,9-dialkyl-silafluorène ;
la solution alcaline est au moins une solution alcaline choisie dans le groupe constitué par une solution aqueuse de NaOH, une solution aqueuse de Na₂CO₃, une solution aqueuse de NaHCO₃ et une solution aqueuse d'hydroxyde de tétraéthylammonium, une quantité molaire de l'alcali dans la solution alcaline est de 2 à 20 fois la quantité molaire du 2,7-bis(4,4,5,5-tétraméthyl-1,3,2-dioxaborolan-yl)-9,9-dialkyl-silafluorène.

9. Procédé de préparation selon la revendication 8, dans lequel le palladium organique est au moins un palladium organique choisi dans le groupe constitué par Pd(PPh₃)₄, Pd(OAc)₂, Pd₂(dba)₃ et Pd(PPh₃)₂Cl₂ ; et le ligand organophosphine est de la tricyclohexylphosphine ou du P(o-Tol)₃, le mélange du palladium organique et du ligand organophosphine est du Pd₂(dba)₃/P(o-Tol)₃.

10. Utilisations du matériau semi-conducteur organique selon l'une quelconque des revendications 1 à 4 dans les domaines des cellules solaires organiques, des transistors à effet de champ organiques, des dispositifs électroluminescents organiques, des mémoires optiques organiques, des dispositifs non linéaires organiques ou des dispositifs laser organiques, etc..
